# EUROPEAN PATENT APPLICATION

(11) **EP 3 202 412 A1**
(43) Date of publication of application: **09.08.2017**
(21) Application number: 14902360.8
(22) Date of filing: 24.09.2014
(51) Int. Cl.: A61K 36/18, A61K 33/44, A61P 1/16, A61P 13/12, B01J 20/20, C01B 32/312

(54) **ORAL ADSORBENT AND METHOD FOR PRODUCING ORAL ADSORBENT**

(71) Applicant: Power Japan Plus Inc., Tokyo 150-0021 (JP)
(72) Inventor: KANI, Dou, Tokyo 150-0021 (JP); NISHINA, Hiroaki, Tokyo 150-0021 (JP); TAKEYA, Kaname, Tokyo 150-0021 (JP); KIDO, Masami, Tokyo 150-0021 (JP); KUBO, Nobuo, Osaka-shi Osaka 541-0043 (JP)
(74) Representative: dompatent von Kreisler Selting Werner - Partnerschaft von Patent- und Rechtsanwälten mbB
(86) International application number: PCT/JP2014/075235
(87) International publication number: WO 2016/046910

(57) **Abstract**

Activated carbon (for example medicinal carbon), which has long been used for adsorbing and removing harmful substances from the digestive system, has the drawback of causing constipation, and the production of a spherical adsorbent carbon that is believed to overcome this drawback from a petroleum-based hydrocarbon requires a complicated production process. These issues can be addressed by an oral adsorbent, a therapeutic or prophylactic agent for kidney disease, and a therapeutic or prophylactic agent for liver disease, containing a fibrous carbonaceous substance obtained by carbonizing cotton fiber. The issues can also be addressed by an oral adsorbent, a therapeutic or prophylactic agent for kidney disease, and a therapeutic or prophylactic agent for liver disease, containing a fibrous carbonaceous substance having a twisted hollow ribbon-like structure.

## Description

### TECHNICAL FIELD

The present invention relates to an oral adsorbent containing a carbonaceous substance. The invention relates particularly to an oral adsorbent that is effective as a therapeutic agent or prophylactic agent for kidney disease, and as a therapeutic agent or prophylactic agent for liver disease.

### BACKGROUND ART

Patients suffering from kidney function or liver function disorders due to all manner of causes including lifestyle diseases tend to accumulate harmful substances within the body that cause the onset of a variety of illnesses.

For example, it is known that patients with chronic kidney failure can exhibit an increase in indoxyl sulfate concentration in the blood serum to about 60 times that of a normal person (Non-Patent Document 1).

The reason that the blood serum indoxyl sulfate concentration increases in patients with chronic kidney failure is described below.

A portion of the tryptophan derived from protein is metabolized in the digestive organs (intestinal tract) by E. coli and the like to form indole, which is subsequently absorbed. Indoxyl sulfate is then produced from this indole in the liver. Indoxyl sulfate is excreted by the kidneys, but in patients with chronic kidney failure, because this excretion pathway does not function normally, indoxyl sulfate accumulates in the blood.

Hemodialysis is widely used for removing these types of harmful substances from the body, but suffers from a number of considerable drawbacks, including requiring a specialist technician and special apparatus, and placing considerable physical, mental, economic and timewise strain on the patient.

As a result, oral adsorbents that can treat or prevent kidney or liver function disorders are now being developed as treatment solutions that do not suffer from these drawbacks.

For example, administration of the oral adsorbent AST-120 (brand name: Kremezin) has been shown to reduce the indoxyl sulfate concentration in blood serum, resulting in an effect of slowing the progression of kidney failure.

In other words, based on multiple clinical trial reports, it is known that the administration of AST-120 to patients suffering chronic kidney failure clearly delays the need for the introduction of artificial dialysis, and tends to result in an improvement in the creatinine concentration in the blood serum observed in patients with kidney failure (Non-Patent Documents 2 and 3).

The abovementioned AST-120 is a spherical adsorbent carbon produced from petroleum-based hydrocarbons, and the ingestion of activated carbon (for example, the medicinal carbon described in The Japanese Pharmacopoeia) for the purpose of adsorbing and removing harmful substances from the digestive system has long been known.

For example, Patent Document 1 discloses that the ingestion of activated carbon has an effect on "bacterial infections such as dysentery, cholera, typhoid fever and food poisoning, as well as indigestion, bowel bloating, chronic gastritis, epilepsy, dizziness, chlorosis and anthrax", and also has an effect on "the ingestion of drugs or poisons or the like".

However, it is known that conventionally used activated carbon tends to cause constipation as a side effect (constipation side effect).

For example, Patent Document 2 states that "a major drawback of activated carbon, which is extremely useful in removing poisons and toxic substances from the body, is that oral administration tends to cause constipation, and therefore a purgative must be jointly administered with the activated carbon", and discloses, as a countermeasure, that "adding chitosan to an activated carbon produced from a plant raw material prevents constipation".

In contrast, AST-120 and a spherical carbonaceous substance disclosed in, for example, Patent Document 1 are said to suppress this constipation side effect.

In other words, it is thought that provided the carbonaceous substance has a prescribed shape, the constipation side effect is less likely to occur, and according to Patent Document 1, the conditions for this prescribed shape require a spherical shape with a diameter of 0.05 to 2 mm, and preferably 0.2 to 1.0 mm.

### PRIOR ART DOCUMENTS

### PATENT DOCUMENTS

Patent Document 1: JP S62-7169 B
Patent Document 2: JP 2002-20297 A
Patent Document 3: JPH10-121337A
Patent Document 4: JP 2002-219357 A

### NON-PATENT DOCUMENTS

Non-Patent Document 1: The Japanese Journal of Nephrology, Vol. XXXII, No. 6 (1990), pp. 65 to 71.
Non-Patent Document 2: Haruhiko Ueda, Nobuhisa Shibahara and others, "Effect of Kremezin in Delaying the Introduction of Dialysis for Chronic Kidney Failure - Investigation of Timing of Administration", Journal of Japanese Society for Dialysis Therapy, Vol. 37, pp. 8 to 33, 2004.
Non-Patent Document 3: Shigeo Wada and Katsuyoshi Matsumuro, "Effect of AST-120 on Diabetic Renal Failure", Progress in Medicine, Vol. 18, No. 3, pp. 483 to 487, 1998.

### SUMMARY OF INVENTION

### TECHNICAL PROBLEM

However, the conventional method of ingesting a purgative together with the activated carbon places an extremely large burden on patients who require long-term administration, such as patients with chronic kidney failure, and the joint ingestion of chitosan or the like also results in an increased amount of drug administration (for example, in Patent Document 2, an amount of chitosan equal to the amount of activated carbon must be ingested), which is also undesirable.

Further, in the case of spherical carbonaceous substances such as those disclosed in Patent Document 1, in order to achieve the specific shape described above, production must be performed via complex and precise production steps, by, for example, using a petroleum-based hydrocarbon (such as petroleum pitch or a phenolic resin) as the raw material, forming very small granules from the raw material, and then performing carbonization and activation.

### SOLUTION TO PROBLEM

In order to address the above problems, in a first aspect of the oral adsorbent of the present invention, the oral adsorbent contains a fibrous carbonaceous substance obtained by carbonizing cotton fiber.

Further, in a second aspect of the oral adsorbent of the present invention, the oral adsorbent contains a fibrous carbonaceous substance having a twisted hollow ribbon-like structure.

### ADVANTAGEOUS EFFECTS OF INVENTION

According to the present invention, by using a fibrous carbonaceous substance obtained by carbonizing cotton fiber, or a fibrous carbonaceous substance having a twisted hollow ribbon-like structure, an effect is obtained that enables production of an oral adsorbent for which the constipation side effect can be suppressed. In other words, the present invention enables the production of an oral adsorbent that requires neither simultaneous administration of a purgative or addition of chitosan or the like, nor the complex production process needed for forming spherical particles having a prescribed diameter, which have typically been necessary to deal with the constipation side effect associated with conventional activated carbon.

### BRIEF DESCRIPTION OF DRAWINGS

FIG. 1 is graph showing creatinine concentration that illustrates the effect of ingesting oral adsorbents in Example 2.
FIG. 2(a) is an electron microscope photograph of a fibrous carbonaceous substance in Example 1.
FIG. 2(b) is an electron microscope photograph of a fibrous carbonaceous substance in Example 1.
FIG. 3 is a schematic diagram illustrating the structure of a cotton fiber.

### DESCRIPTION OF EMBODIMENTS

As a result of intensive investigation, the inventors of the present invention has discovered that a fibrous carbonaceous substance obtained by carbonizing cotton fibers is effective as an oral adsorbent for which the constipation side effect is suppressed.

The carbonized substance obtained by processing natural cotton fibers is itself a known substance. For example, Patent Document 3 discloses "carbon fiber obtained by carbonizing raw cotton and/or a fibrous bundle containing mainly raw cotton having a fiber length of not more than 20 mm", and Patent Document 4 discloses "carbonized cotton obtained by calcining cotton".

However, with respect to the applications of these substances, Patent Document 3 discloses that "when brought into contact with colored wastewater or industrial wastewater having a high concentration of organic matter, the effects of decolorization, BOD reduction, and removal of harmful gases are seen", whereas Patent Document 4 discloses "means for adsorbing and removing harmful gases, liquids, microorganisms and odors".

In other words, as far as the inventors of the present invention are aware, the use of a fibrous carbonaceous substance obtained by carbonizing cotton fibers as an oral adsorbent has not previously been proposed, and there have certainly been no previous proposals nor mentions of the fact that a fibrous carbonaceous substance obtained by carbonizing cotton fibers could be expected to have an effect in treating or preventing kidney disease and treating or preventing liver disease, or the fact that such a fibrous carbonaceous substance obtained by carbonizing cotton fibers could act as an oral adsorbent which, despite not having the prescribed shape conventionally thought necessary, was capable of suppressing the constipation side effect.

The fibrous carbonaceous substance used in the present invention is described below.

The cotton fibers that act as the raw material for the fibrous carbonaceous substance used in the present invention refer to fibers collected from the seeds of the cotton plant (the burst seed pods are referred to as raw cotton), and there are no particular limitations on the type or shape of the fibers, provided they are of a purity suitable for ingestion. In other words, cotton is grown throughout the world, and examples of known cotton types include Asiatic cotton, Indian Desi cotton, Pakistan Desi cotton, upland cotton, American cotton, Soviet cotton, Australian cotton, Chinese cotton, Sea Island cotton, Egyptian cotton, Peruvian cotton, Indian cotton, Sudanese cotton and supima cotton, whereas the form of the fibers may be the raw cotton itself, clumps of fibers (such as medical cotton wool), or processed products such as nonwoven fabrics or cotton fabric made from cotton.

### <Production of Fibrous Carbonaceous Substance>

The fibrous carbonaceous substance used in the present invention is produced by the extremely simple steps of a carbonization step and a crushing step.

### <Carbonization Step>

The fibrous carbonaceous substance used in the present invention is produced by heating and carbonizing the cotton fibers in an inert gas atmosphere, but there are no particular limitations on the carbonization conditions. Interestingly, compared with conventional methods for carbonizing cotton fibers for use in adsorption, a favorable oral adsorbent can be produced even when carbonization is performed at higher temperatures.

For example, Patent Document 3 discloses that the maximum temperature for the carbonization is preferably not more than 700°C, whereas Patent Document 4 discloses that "the cotton is heated and calcined at 200 to 500°C".

The fibrous carbonaceous substance used in the present invention may, of course, be produced by carbonization at conventional temperatures, but it has been confirmed that an oral adsorbent containing a fibrous carbonaceous substance produced by carbonization at a maximum temperature of at least 1,000°C but not more than 1,600°C is capable of inducing a reduction in the creatinine concentration in blood serum, or in other words, can be used effectively as a therapeutic or prophylactic agent for kidney disease or liver disease.

Further, it has been confirmed that the fibrous carbonaceous substance used in the present invention can be used to produce an oral adsorbent that is effective as a therapeutic or prophylactic agent for kidney disease or liver disease even without performing the type of activation step that is typically required in activated carbon production.

The activation step describes a step of heating the carbonized product obtained following the carbonization step, for a fixed period at a high temperature (600 to 1,200°C) in a gaseous atmosphere such as an atmosphere of steam or carbon dioxide.

### <Crushing Step>

The fibrous carbonaceous substance used in the present invention is crushed prior to use to obtain a fiber length suitable for ingestion.

The crushing may be performed using a mechanical crushing device (such as a ball mill or blade mill), or using a pneumatic crushing device (such as a jet mill). The crushing step is usually performed after the carbonization step, but the raw material cotton fibers may be crushed prior to the carbonization.

There are no particular limitations on the fiber length following crushing, provided the size is suitable for ingestion. A sieve or an airflow classification device may be used to adjust the fiber length distribution. The fiber length distribution is typically from at least 10 µm to not more than 2 mm, and is preferably from at least 50 µm to not more than 1 mm.

### <Shape of Fibrous Carbonaceous Substance>

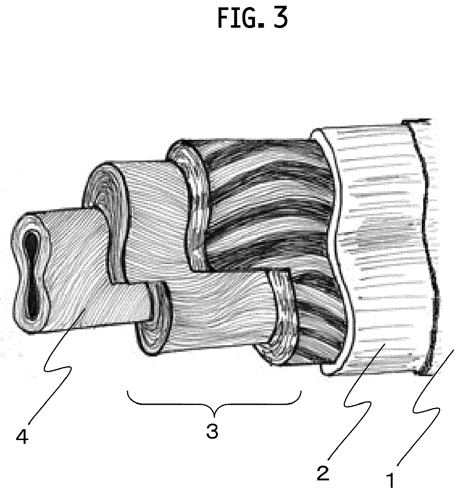

The cotton fibers have an elongated, twisted, hollow ribbon-like shape, and have a three dimensional layered structure composed, form the outside surface, of a cuticular layer, a first cellulose layer, a second cellulose layer, and the lumen (FIG. 3: extract from the home page of "Oyasumidokoro 'Tokiwasarashi' Cotton Shop Takita").

It is thought that when the cotton fibers are heated, the cuticular layer is burned, but the cellulose layers and the lumen are carbonized with their shape substantially retained, meaning the carbonaceous substance that is the carbonized product has a twisted hollow ribbon-like three dimensional structure.

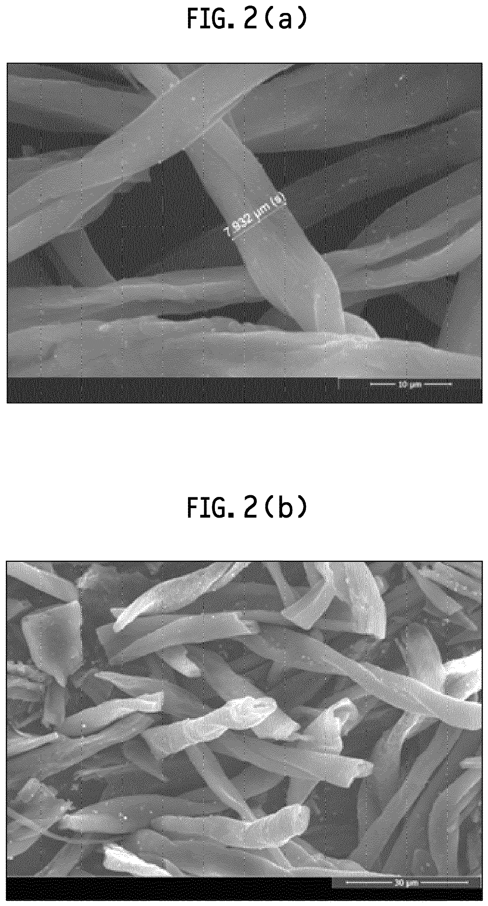

FIG. 2(a) and FIG. 2(b) show electron microscope photographs of a fibrous carbonaceous substance obtained by carbonizing cotton fibers, that is a which represents the raw material for the oral adsorbent of the present invention (see the examples). The three dimensional structure is not entirely clear in some portions, but a twisted hollow ribbon-like structure can be confirmed. The measurements were performed using a Versa 3D HiVac manufactured by FEI Company.

The mechanism by which the fibrous carbonaceous substance used in the present invention suppresses the constipation side effect is not entirely clear. However, the inventors of the present invention think that the shape of the fibrous carbonaceous substance obtained by carbonizing the cotton fibers is one reason.

### <Structure of Oral Adsorbent>

The fibrous carbonaceous substance used in the present invention may be ingested in a crushed state together with water or the like, but is usually processed to form tablets or capsules for ingestion.

During processing, a binder and/or a sweetener (for example, reduced maltose, which can also act as a binder) or the like may be added to facilitate ingestion, and a food additive (such as a vitamin or a mineral) and the like may also be added, provided it can be thought that the adsorption function of the fibrous carbonaceous substance are not impaired.

### EXAMPLES

The present invention is described below in further detail using examples.

### <Example 1: Production of Fibrous Carbonaceous Substance>

A commercially available uncolored cotton cloth was placed in a graphite crucible, and a carbonization treatment was performed by heating the cloth to a maximum temperature of 1,400°C under an argon atmosphere.

The thus obtained carbonized product was then crushed for about one minute in an agate mortar.

FIG. 2(a) and FIG. 2(b) show examples of electron microscope photographs of the fibrous carbonaceous substance obtained in this example. The diameter of the fibrous carbonaceous substance determined from these electron microscope photographs was at least 1 µm but not more than 10 µm, and the fiber length was at least 10 µm but not more than 2 mm.

Further, the total pore volume was 6.27 × 10⁻² mL/g, the BET specific surface area was 1.10 m²/g, and the average pore diameter was 3.80 nm (the total pore volume and the BET specific surface area were measured using a Belsorp-mini II manufactured by MicrotracBEL Corporation, and the average pore diameter was calculated from these values).

### <Example 2: Effects of Oral Adsorbent>

FIG. 3 illustrates the results of ingesting the oral adsorbent of the present invention.

The recipients totaled 11 people, composed of five people from a so-called prechronic kidney disease group (CKD) and six people from a pre-diabetes mellitus group (DM).

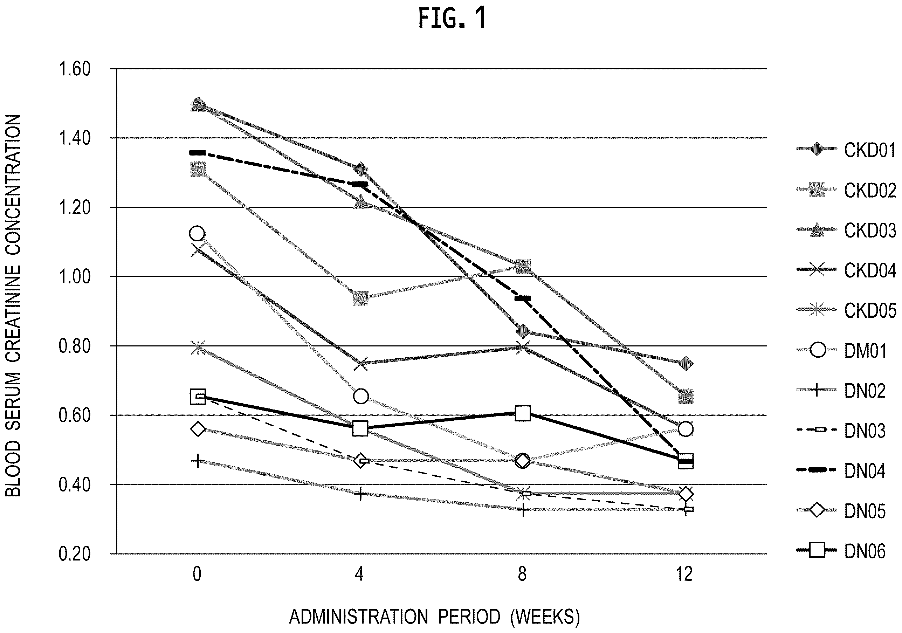

To each of the above 11 recipients, 3 g of the fibrous carbonaceous substance produced in Example 1 were administered for ingestion per day for a period of three months, and observation of the creatinine concentration in the blood serum over time revealed a decrease in the creatinine concentration in all 11 recipients, as illustrated in FIG. 1. It was found that the oral adsorbent in the present invention had an effect in moderating the symptoms of chronic kidney disease. In other words, a slowing effect on the progression of kidney failure and a delay in the need for the introduction of artificial dialysis can be expected.

Further, among the above 11 recipients, not a single person complained of constipation symptoms.

In FIG. 1, the vertical axis (creatinine concentration in blood serum) is normalized relative to a value of 1 for the average blood serum creatinine concentration across the 11 recipients prior to starting administration.

### INDUSTRIAL APPLICABILITY

The oral adsorbent of the present invention can be expected to provide a therapeutic or improvement effect for gastrointestinal disease or drug poisoning, similarly to conventional medicinal carbon.

Further, the oral adsorbent of the present invention can be used as an oral adsorbent for treating or preventing kidney disease, or used as an oral adsorbent for treating or preventing liver disease.

### DESCRIPTION OF THE REFERENCE SIGNS

1: Cuticular layer
2: First cellulose layer
3: Second cellulose layer
4: Lumen

## Claims

1. An oral adsorbent comprising a fibrous carbonaceous substance obtained by carbonizing cotton fiber.

2. An oral adsorbent comprising a fibrous carbonaceous substance having a twisted hollow ribbon-like structure.

3. The oral adsorbent according to Claim 1 or Claim 2, wherein a diameter of the fibrous carbonaceous substance is at least 1 µm but not more than 10 µm.

4. The oral adsorbent according to any one of Claim 1 to Claim 3, wherein a specific surface area of the fibrous carbonaceous substance measured by a BET method is at least 1 m²/g but not more than 500 m²/g.

5. The oral adsorbent according to any one of Claim 1 to Claim 4, wherein a fiber length of the fibrous carbonaceous substance is at least 10 µm but not more than 2 mm.

6. A method for producing a fibrous carbonaceous substance used in the oral adsorbent according to any one of Claim 1 to Claim 5, wherein a maximum temperature during carbonization of the cotton fiber is at least 1,000°C but not more than 1,600°C.

7. A therapeutic agent for kidney disease comprising the oral adsorbent according to any one of Claim 1 to Claim 5 as an active ingredient.

8. A prophylactic agent for kidney disease comprising the oral adsorbent according to any one of Claim 1 to Claim 5 as an active ingredient.

9. A therapeutic agent for liver disease comprising the oral adsorbent according to any one of Claim 1 to Claim 5 as an active ingredient.

10. A prophylactic agent for liver disease comprising the oral adsorbent according to any one of Claim 1 to Claim 5 as an active ingredient.
